# EUROPÄISCHE PATENTSCHRIFT

(11) **EP 2 225 220 B1**
(45) Veröffentlichungstag und Bekanntmachung des Hinweises auf die Patenterteilung: **22.05.2013**
(21) Anmeldenummer: 08863798.8
(22) Anmeldetag: 10.12.2008
(51) Int. Cl.: C07D 313/04

(54) **Verfahren zur Herstellung von epsilon-Caprolacton**
Method for producing epsilon-caprolactone
Procédé de fabrication d'epsilon-caprolactone

(30) Priorität: 21.12.2007 EP 07150291
(43) Veröffentlichungstag der Anmeldung: 08.09.2010
(73) Patentinhaber: BASF SE, 67056 Ludwigshafen (DE)
(72) Erfinder: PINKOS, Rolf, 67098 Bad Dürkheim (DE); TEBBEN, Dr. Gerd-Dieter, 68239 Mannheim (DE); BREUNINGER, Daniel, 67240 Bobenheim-Roxheim (DE); SIRCH, Tilman, 67105 Schifferstadt (DE); BAUDUIN, Christophe, 68723 Plankstadt (DE); GUIXA GUARDIA, Maria, 68163 Mannheim (DE); KRUG, Thomas, 67550 Worms (DE)
(86) Internationale Anmeldenummer: PCT/EP2008/067180
(87) Internationale Veröffentlichungsnummer: WO 2009/080504

(56) Entgegenhaltungen:
- WO-A-97/31883
- DE-A1- 3 823 213

## Beschreibung

Die Erfindung betrifft ein Verfahren zur Herstellung von ε-Caprolacton aus 6-Hydroxycapronsäureester in der Gasphase in Gegenwart von Aktivkohle als Katalysator und anschließender Destillation.

ε-Caprolacton bzw. die daraus durch Polyaddition hergestellten Polycaprolactone dienen zur Herstellung von Polyurethanen.

Die wässrigen Lösungen von Carbonsäuren, die bei der Oxidation von Cyclohexan zu Cyclohexanol und Cyclohexanon wie es in Ullmann's Encyclopedia of Industrial Chemistry, 5. Ed., 1987, Vol. A8, Seite 219 beschrieben ist, als Nebenprodukte entstehen, im folgenden Dicarbonsäurelösung (DCL) genannt, enthalten (berechnet wasserfrei in Gew.-%) im allgemeinen zwischen 10 und 40% Adipinsäure, zwischen 10 und 40 % 6-Hydroxycapronsäure, zwischen 1 und 10% Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und gamma-Butyrolacton genannt.

Auch die Herstellung von Caprolacton aus DCL ist z.B. aus DE 1 618 143 bereits beschrieben worden. Dabei wird entwässerte DCL mit Phosphorsäure thermisch umgesetzt und ein Gemisch aus Dicarbonsäuren, Caprolacton sowie eine Vielzahl anderer Komponenten fraktioniert. Der Sumpf fällt dabei z.T. fest und schwerlöslich an. Das Caprolacton hat aber auch nach weiterer destillativer Aufarbeitung nur eine 98 %ige Reinheit.

Ferner ist in DE 38 23 213 beschrieben, 6-Hydroxycapronsäureester in der Gasphase in Gegenwart oxidischer Katalysatoren und eines inerten Trägergases zu Caprolacton umzusetzen. Nachteilig gegenüber dem erfindungsgemäßen Verfahren ist der Einsatz von oxidischen Katalysatoren in der Gasphase, da sie zu einer erhöhten Nebenproduktbildung neigen.

Weiterhin ist in WO 97/31883 ein Verfahren beschrieben zur Herstellung von 1,6-Hexandiol und ε-Caprolacton aus einem Adipinsäure, 6-Hydroxycapronsäure und geringe Mengen 1,4-Cyclohexandiole enthaltenden Carbonsäuregemisch, das als Nebenprodukt der Oxidation von Cyclohexan zu Cyclohexanon/Cyclohexanol mit Sauerstoff oder Sauerstoff enthaltenden Gasen und durch Wasserextraktion des Reaktionsgemisches erhalten wird, welches man mit einem niedermolekularen Alkohol zu den entsprechenden Carbonsäureestern verestert, das erhaltene Veresterungsgemisch in einer ersten Destillationsstufe von überschüssigen Alkohol und Leichtsiedern befreit, aus dem Sumpfprodukt in einer zweiten Destillationsstufe eine Auftrennung in einen von 1,4-Cyclohexandiolen im wesentlichen freie Esterfraktion und zumindest den größeren Teil der Cyclohexandiole enthaltenden Fraktion vornimmt, durch eine dritte Destillationsstufe eine im wesentlichen 6-Hydroxycapronsäure enthaltende Fraktion (Stufe 12) gewinnt und in der Gas- oder Flüssigphase zu ε-Caprolacton, cyclisiert.

Da sich der Siedebereich von Adipinsäureester und 6-Hydroxycapronsäureester kaum unterscheidet, kann man im Allgemeinen beide Stoffe nur unter extrem hohem destillativen Aufwand ohne den jeweils anderen gewinnen, z. B. durch Verwendung von Kolonnen mit sehr hohen Trennstufenzahlen und einem entsprechend hohen Energieaufwand, oder durch Zugabe eines Fremdstoffes, der einen Siedepunkt zwischen den beiden Estern aufweist.

Um den Trennaufwand zu vermindern und um reinen 6-Hydroxycapronsäureester zu gewinnen, wurde die destillative Trennung der beiden C₆-Ester in der dritten Destillationsstufe gemäß WO 97/31883 bisher so durchgeführt, dass der zum 1,6-Hexandiol zu hydrierende Adipinsäurediester noch 0,2 bis 7 Gew.-% 6-Hydroxycapronsäureester bezogen auf den Anteil an 6-Hydroxycapronsäureester, der zusammen mit Adipinsäureester in die Hydrierung zu 1,6-Hexandiol geht, enthielt. Bei hohem Bedarf an 1,6-Hexandiol kann unter weiterer Verringerung des Trennaufwands auch noch mehr 6-Hydroxycapronsäureester zusammen mit Adipinsäurediester abgetrennt und zu 1,6-Hexandiol hydriert werden. Der 6-Hydroxycapronsäureester-Gehalt der Dicarbonsäurelösung wurde daher bisher nie vollständig für die Caprolacton-Herstellung genutzt.

Ist die Nutzung von mehr als 50%, bevorzugt > 80 %, besonders bevorzugt > 90% des Anteils des 6-Hydroxycapronsäureesters zur Caprolactonherstellung gewünscht, ohne dass ein extrem großer destillativer Aufwand oder die Zugabe eines Fremdstoffes erfolgt, so muss die Cyclisierung des 6-Hydroxycapronsäureester-Strom in Gegenwart von Adipinsäureester in Mengen von mindestens 0,5 Gew.-%, im Allgemeinen > 1 % bis zu 25 Gew.-%, bezogen auf den Gesamtfeed zur Caprolactonsynthese, ohne Nachteile möglich sein.

WO 97/31883 empfiehlt die Herstellung von Caprolacton in der Flüssigphase. Entsprechend des in dieser Anmeldung enthaltenen Vergleichsbeispiels 1 wird für die Cyclisierung in der Flüssigphase in Gegenwart des von 5 Gew.-% Adipinsäureesters, bezogen auf den 6-Hydroxycapronsäureesters, jedoch ein deutlicher Rückgang der Ausbeute an Caprolacton beobachtet.

Dieser Ausbeuterückgang ist auf Polymerisationsnebenreaktionen in der ε-Caprolacton-Cyclisierung zurückzuführen. In Gegenwart von Katalysatoren können aus Adipinsäurediestern und 6-Hydroxycapronsäureestern Dimere, Oligomere oder Polymere entstehen. Aus Adipinsäuredimethylester und 6-Hydroxycapronsäuremethylester kann sich z. B. der dimere Ester CH₃OOC-(CH₂)₄-COO-(CH₂)₅-COOCH₃ bilden, der unter Einbau weiterer 6-Hydroxycapronsäureester Oligomere oder Polymere bilden kann. Diese Dimere, Oligomere oder Polymere stellen zwar noch durch Hydrierung verwertbare Verbindungen für 1,6-Hexandiol dar, jedoch ist bei Reaktionen in der Gasphase, die Gefahr von Ablagerungen dieser hochsiedenden Komponenten, auf dem Cyclisierungskatalysator groß, so dass mit einer sehr verkürzten Katalysatorstandzeit gerechnet werden müsste.

Weiterhin war aus EP-A 251 111 bekannt, dass Adipinsäurediester in Gegenwart von Katalysatoren zu Cyclopentanonen umgewandelt werden und damit nicht mehr für andere Anwendungen wie beispielsweise die Umsetzung zu 1,6-Hexandiol zur Verfügung stehen.

Es bestand daher die Aufgabe, ein Verfahren zur Herstellung von Caprolacton in einer Reinheit von mehr als 99% ausgehend von 6-Hydroxycapronsäureestern oder deren Gemischen mit Adipinsäurediestern bereitzustellen, bei dem eine Verringerung des Trennaufwands und die Nutzung von mehr als 50%, bevorzugt > 80 %, besonders bevorzugt > 90% des Anteils des 6-Hydroxycapronsäureesters zur Caprolacton-Herstellung einhergehen und durch Vermeidung von Polymerisationsnebenreaktionen in der ε-Caprolacton-Cyclisierung gute Katalysatorstandzeiten und CaprolactonAusbeuten und Selektivitäten erreicht werden. Zudem sollte möglichst wenig Adipinsäureester umgesetzt werden, da dieser nach Abtrennung von Caprolacton möglichst noch anderen Anwendungen zur Verfügung stehen sollte.

Diese Aufgabe wird gelöst durch ein Verfahren zur Herstellung von ε-Caprolacton in einer Reinheit über 99 %, dadurch gekennzeichnet, dass 6-Hydroxycapronsäureester enthaltend 0 bis 40 Gew.-% Adipinsäurediester in der Gasphase bei 150 bis 450 °C in Gegenwart von Aktivkohle als Katalysator cyclisiert und aus dem Cyclisierungsprodukt durch Destillation ε-Caprolacton gewonnen wird.

Vorteilhaft ist das erfindungsgemäße Verfahren, wenn die 6-Hydroxycapronsäure 0,5 bis 40 Gew.-% Adipinsäurediester enthält.

Vorteilhaft ist das erfindungsgemäße Verfahren, wenn der 6-Hydroxycapronsäureester enthaltend 0 bis 40 Gew.-% Adipinsäurediester durch katalytische Hydrierung von Adipinsäurediestern oder Eduktströmen, die diese Ester als wesentliche Bestandteile enthalten, Destillation des Hydrieraustrags und Abtrennung des Hexandiols, gewonnen wird.

Vorteilhaft ist das erfindungsgemäße Verfahren, wenn ein Adipinsäure-, 6-Hydroxycapronsäure- und bis zu 5 Gew.-% 1,4-Cyclohexandiole enthaltendes Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhältlich ist, mit einem Alkohol der 1 bis 12C-Atome enthält zu den entsprechenden Carbonsäureestern verestert wird, das so erhaltene Veresterungsgemisch in mindestens einer Destillationsstufe so aufgetrennt wird, dass man den 0 bis 40 Gew.-% Adipinsäurediester enthaltenden 6-Hydroxycapronsäureester-Strom erhält.

Vorteilhaft ist das erfindungsgemäße Verfahren, wenn zur Herstellung des 6-Hydroxycapronsäuremethylester enthaltend 0 bis 40 Gew.-% Adipinsäuredimethylester
- das erhaltene Veresterungsgemisch in einer ersten Destillationsstufe von überschüssigem Methanol und Leichtsiedern befreit wird,
- aus dem Sumpfprodukt in einer zweiten Destillationsstufe eine Auftrennung in eine Esterfraktion mit einem Gehalt von weniger als 20 % 1,4 Cyclohexandiole bezogen auf den ursprünglichen Gehalt an 1,4-Cyclohexandiolen des Estergemischs und eine mit mehr als 80 % 1,4-Cyclohexandiole bezogen auf den ursprünglichen Gehalt an 1,4-Cyclohexandiolen des Estergemischs enthaltende Fraktion durchgeführt wird,
- der 0 bis 40 Gew.-% Adipinsäuredimethylester enthaltenden 6-Hydroxycapronsäuremethylester-Strom von der Esterfraktion in einer dritten Destillationsstufe abgetrennt wird.

Vorteilhaft ist das erfindungsgemäße Verfahren, wenn in Gegenwart eines inerten Trägergases ausgewählt aus Gruppe von Stickstoff, Kohlendioxid, Wasserstoff und Edelgasen cyclisiert wird.

Vorteilhaft ist das erfindungsgemäße Verfahren, wenn bei 150 bis 450 °C cyclisiert wird.

Der im erfindungsgemäße Verfahren eingesetzte 6-Hydroxycapronsäureester enthält 0 bis 40 Gew.-%, bevorzugt 0,5 bis 25 Gew.-%, besonders bevorzugt 0,6 bis 15 Gew.-% Adipinsäurediester und wird in der Gasphase bei 150 bis 450°C in Gegenwart von Aktivkohle als Katalysator zum ε-Caprolacton cyclisiert und aus dem Cyclisierungsprodukt wird anschließend durch Destillation das ε-Caprolacton gewonnen.

Als für die Veresterung eingesetzte Alkohole des 6-Hydroxycapronsäureesters und des Adipinsäureesters kommen in der Regel Alkanole mit 1 bis 12 C-Atomen, Cycloalkanole mit 5 bis 7 C-Atomen, Aralkanole mit 7 bis 8 C-Atomen oder Phenole mit 6 bis 8 C-Atomen in Betracht. Dabei können Methanol, Ethanol, Propanol, iso-Propanol, n- oder i-Butanol oder auch n-Pentanol oder i-Pentanol oder Gemische der Alkohole, bevorzugt aber Alkohole mit 1 bis 4 C-Atomen, besonders bevorzugt Methanol verwendet werden. Auch Diole wie Butandiol oder Pentandiol kommen prinzipiell in Betracht. Die Estergruppen in den 6-Hydroxycapronsäureestern und den Adipinsäurediestern können gleich oder verschieden sein, bevorzugt sind sie jedoch gleich. Das besonders bevorzugte Edukt ist 6-Hydroxycapronsäuremethylester enthaltend 0 bis 40 Gew.-% Adipinsäuredimethylester.

Die Herstellung des Edukts des erfindungsgemäßen Verfahrens, des 6-Hydroxycapronsäureesters enthaltend 0 bis 40 Gew.-% Adipinsäurediester, kann auch gemäß DE-A 197 50 532, auf die hier ausdrücklich Bezug genommen wird und hier als inkorporiert gelten soll, erfolgen.

Gemäß DE-A 197 50 532 wird 6-Hydroxycapronsäureester enthaltend 0 bis 40 Gew.-% Adipinsäurediester durch katalytische Hydrierung von Adipinsäurediestern oder Eduktströmen, die diese Ester als wesentliche Bestandteile enthalten, Destillation des Hydrieraustrags und Abtrennung des Hexandiols gewonnen.

Dabei wird die Hydrierung bevorzugt in der Flüssigphase durchgeführt. Als Hydrierkatalysatoren finden in diesem Verfahren im Allgemeinen heterogene, aber auch homogene, zur Hydrierung von Carbonylgruppen geeignete Katalysatoren Verwendung. Sie können sowohl fest angeordnet, als auch mobil, z. B. in einem Wirbelbettreaktor, eingesetzt werden. Beispiele hierfür sind z. B. in Houben-Weyl, Methoden der Organischen Chemie, Band IV/1c, S. 16 bis 26 beschrieben.

Von den zu verwendenden Hydrierkatalysatoren sind solche bevorzugt, die ein oder mehrere Elemente der Gruppe Ib, Vlb, Vllb und Vlllb, sowie IIIa, IVa und Va des Periodensystems der Elemente, insbesondere Kupfer, Chrom, Rhenium, Kobalt, Rhodium, Nickel, Palladium, Eisen, Platin, Indium, Zinn und/oder Antimon enthalten. Besonders bevorzugt sind Katalysatoren, die Kupfer, Kobalt und/oder Rhenium enthalten.

Weiterhin kann die Herstellung des 6-Hydroxycapronsäureesters enthaltend 0 bis 40 Gew.-% Adipinsäurediester gemäß WO 97/31 883, auf die hier ausdrücklich Bezug genommen wird und hier als inkorporiert gelten soll, erfolgen.

Die Herstellung des 6-Hydroxycapronsäureester enthaltend 0 bis 40 Gew.-% Adipinsäurediester erfolgt nach WO 97/31 883 derart, dass ein Adipinsäure, 6-Hydroxycapronsäure und geringe Mengen 1,4-Cyclohexandiole enthaltendes Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhältlich ist, mit einem niedermolekularen Alkohol zu den entsprechenden Carbonsäureestern verestert wird, und das so erhaltene Veresterungsgemisch in mindestens einer Destillationsstufe aufgetrennt wird.

In einer bevorzugten Ausführungsform wird 6-Hydroxycapronsäuremethylester enthaltend 0 bis 40 Gew.-% Adipinsäuredimethylester erhalten, in dem
- das erhaltene Veresterungsgemisch in einer ersten Destillationsstufe von überschüssigem Methanol und Leichtsiedern befreit wird,
   aus dem Sumpfprodukt in einer zweiten Destillationsstufe eine Auftrennung in eine Esterfraktion mit einem Gehalt von weniger als 20 % 1,4 Cyclohexandiole bezogen auf den ursprünglichen Gehalt an 1,4-Cyclohexandiolen des Estergemischs und eine mit mehr als 80 % 1,4-Cyclohexandiole bezogen auf den ursprünglichen Gehalt an 1,4-Cyclohexandiolen des Estergemischs enthaltende Fraktion durchgeführt wird.
- der 0 bis 40 Gew.-% Adipinsäuredimethylester enthaltende 6-Hydroxycapronsäuremethylester-Strom von der Esterfraktion in einer dritten Destillationsstufe abgetrennt wird.

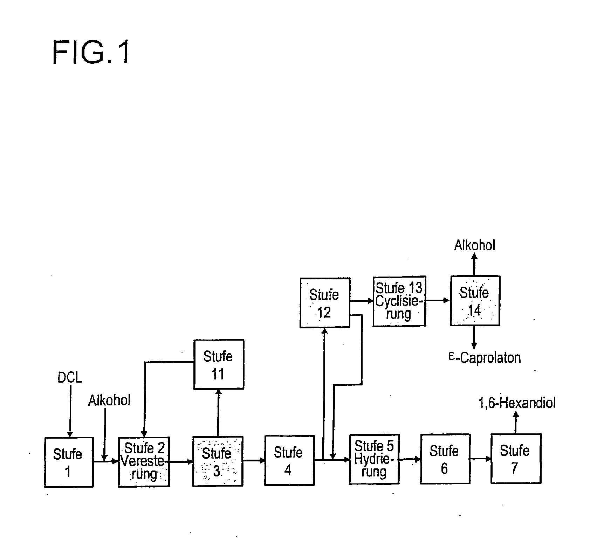

Zum besseren Verständnis wird das Verfahren zur Herstellung von ε-Caprolacton entsprechend WO 97/31883 gemäß Figur 1 erläutert, in der die einzelnen Verfahrensschritte in weitere Stufen aufgeschlüsselt sind, wobei die Stufen 2, 3, 4 sowie 12, 13 und 14 für das Verfahren zur Herstellung von ε-Caprolacton essentiell sind und die Stufen 3 und 4 auch zusammengefasst werden können.

Die Dicarbonsäurelösung (DCL) ist im Allgemeinen eine wässrige Lösung mit einem Wasseranteil von 20 bis 80 Gew.-%. Da eine Veresterungsreaktion eine Gleichgewichtsreaktion darstellt, bei der Wasser entsteht, ist es sinnvoll, insbesondere bei Veresterung mit z. B. Methanol, vorhandenes Wasser vor der Reaktion zu entfernen, vor allem wenn während der Veresterungsreaktion Wasser nicht, z. B. nicht azeotrop, entfernt werden kann. Die Entwässerung in Stufe 1 kann z. B. mit einem Membransystem erfolgen, oder bevorzugt durch eine Destillationsapparatur, bei der bei 10 bis 250 °C, bevorzugt 20 bis 200 °C, besonders bevorzugt 30 bis 200 °C und einem Druck von 1 bis 1500 mbar, bevorzugt 5 bis 1100 mbar, besonders bevorzugt 20 bis 1000 mbar Wasser über Kopf und C₃-C₇ -Monocarbonsäuren, Dicarbonsäuren und 1,4-Cyclohexandiole über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, dass das Wasser einen Carbonsäuregehalt von < 0,5 Gew.-% enthält oder man kann die in der DCL enthaltenen C₁-C₃-Monocarbonsäuren - mit einem Ameisensäure von > 60 Gew.- % an diesen Carbonsäuren - zu mehr als > 80 % mit dem Wasser abdestillieren, damit diese in der Veresterung keinen Veresterungsalkohol binden.

Den Carbonsäureestern aus der Stufe 1 wird Alkohol ROH mit 1 bis 10 C-Atomen zugemischt. Dabei können Methanol, Ethanol, Propanol oder iso-Propanol oder Gemische der Alkohole, bevorzugt aber Methanol einerseits oder C₄- und höhere Alkohole, insbesondere mit 4 bis 8 C-Atomen und bevorzugt n- oder i-Butanol oder auch n-Pentanol oder i-Pentanol andererseits verwendet werden. Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400 °C, bevorzugt 70 bis 300 °C, besonders bevorzugt 90 bis 200 °C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z. B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z. B. saure und supersaure Metalloxide wie SiO₂, Al₂O₃, SnO₂, ZrO₂, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z. B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10. Dabei müssen nicht alle im System vorhandenen Carboxylgruppen als Ester des eingesetzten Alkohols vorliegen, sondern ein Teil kann in Form von dimeren oder oligomeren Estern mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, - Carbonate, -Hydroxyde oder -Alkoholate, oder Amine in Substanz oder in dem Veresterungsalkohol gelöst verwendet. Es kann jedoch auch mit basischen lonentauschern neutralisiert werden.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150 °C, bevorzugt 15 und 90 °C und insbesondere 25 und 75 °C der überschüssige Veresterungsalkohole ROH, Wasser sowie entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren wie Adipinsäure und Glutarsäure, Hydroxycarbonsäuren wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 4 Gew.-% im Estergemisch zuzulassen. Die Sumpftemperaturen betragen 70 bis 250 °C, bevorzugt 80 bis 220 °C, besonders bevorzugt 100 bis 190 °C.

Der Strom aus Stufe 3, der einen Restgehalt von Wasser und Veresterungsalkohol ROH von jeweils unter 5 Gew.-% enthält, wird in die Stufe 4 eingespeist. Die Kolonne wird bei Temperaturen von 10 bis 300 °C, bevorzugt 20 bis 270 °C, besonders bevorzugt 30 bis 250 °C und Drucken von 1 bis 1 000 mbar, bevorzugt 5 bis 500 mbar, besonders bevorzugt 10 bis 200 mbar betrieben.

Die Kopffraktion enthält bis zu 100 Gew.-% an Restwasser und Restalkohol ROH, Estern des Alkohols ROH mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestern mit Dicarbonsäuren wie Adipinsäure, Glutarsäure und Bernsteinsäure, Cyclohexandiolen, Caprolacton und Valerolaceton.

Die genannten Komponenten können zusammen über Kopf abgetrennt oder in einer weiteren bevorzugten Ausführungsform in der Kolonne der Stufe 4 in einen Kopfstrom, bis zu 99 Gew.-% an Restwasser und Restalkohol sowie die oben erwähnten Bestandteile mit 3 bis 5 C-Atomen enthalten und einen Seitenstrom, der bis zu 99 Gew.-% die oben erwähnten Bestandteile der C₆-Ester enthält, aufgetrennt werden. Der die Ester der C₆-Säuren enthaltende Strom, entweder als Gesamt-Kopfstrom oder als Seitenstrom kann dann, je nachdem wie viel Caprolacton hergestellt werden soll, gemäß dem nach WO 97/31883 bevorzugten Verfahren nur zum Teil oder als Gesamtstrom in die Stufe 12 eingespeist werden.

Die schwersiedenden Komponenten, also jene, die gleich oder höher sieden als die 1,4-Cyclohexandiole des Stromes aus Stufe 4, enthaltend dimere oder oligomere Ester, Cyclohexandiole sowie nicht näher definierte z. T. polymere Bestandteile der DCL, werden über den Abtriebsteil der Kolonne der Stufe 4 abgetrennt. Diese können entweder verbrannt werden oder in einer bevorzugten Ausführungsform zur so genannten Umesterung in die in WO 97/31883 beschriebene Stufe 8 gelangen.

Die Stufen 3 und 4 können, insbesondere wenn nur kleinere Mengen verarbeitet werden, zusammengefasst werden. Bevorzugt werden die Stufen 3 und 4 dann zusammengelebt, wenn weniger als 100 t im Jahr hergestellten werden. Dazu kann beispielsweise in einer absatzweise durchgeführten fraktionierten Destillation der C₆-Esterstrom gewonnen werden.

Für die Caprolactonherstellung wird der bis zu 99 Gew.-% an Ester der C₆-Säuren enthaltende Strom aus der Stufe 4 eingesetzt. Dazu wird dieser Strom in Stufe 12, einer Destillationskolonne, in einen bis zu 98 Gew.-% an Adipinsäurediester enthaltenden Strom über Kopf und einen überwiegend 6-Hydroxycapronsäureester enthaltenden Strom über Sumpf aufgetrennt. Die Kolonne wird bei Drücken von 1 bis 500 mbar, bevorzugt 5 bis 350 mbar, besonders bevorzugt 10 bis 200 mbar und Sumpftemperaturen von 80 bis 250 °C, bevorzugt 100 bis 200 °C, besonders bevorzugt 110 bis 180 °C betrieben. Die Kopftemperaturen stellen sich dabei entsprechend ein.

Wichtig für eine hohe Reinheit und hohe Ausbeute an Caprolacton ist die Abtrennung der 1,2-Cyclohexandiole vom Hydroxycapronsäureester, da diese Komponenten Azeotrope miteinander bilden. Es war in dieser Stufe 12 nicht vorauszusehen, dass die Trennung der 1,2-Cyclohexandiole und des Hydroxycapronsäureesters vollständig gelingt, vor allem wenn als Ester der bevorzugte Methylester eingesetzt wird.

Es kann vorteilhaft sein, in der Stufe 12 zusammen mit dem Adipinsäurediester auch etwas Hydroxycapronsäureester abzutrennen. Die Gehalte des Adipinsäureesters an Hydroxycapronsäureester liegen, wenn der Adipinsäurediester zu 1,6-Hexandiol hydriert werden soll, dabei vorteilhaft zwischen 0,2 und 20 Gew.-%. Je nach Alkoholkomponente der Ester wird dieser Anteil Hydroxycapronsäureester zusammen mit dem Adipinsäurediester über Kopf (z. B. Methylester) oder über Sumpf (z. B. Butylester) abgetrennt.

Der 0 bis 40 Gew.-% Adipinsäurediester aufweisende 6-Hydroxycapronsäureester enthaltende Strom wird in der Gasphase zu Alkohol und Caprolacton umgesetzt. Diese Gemische aus 6-Hydroxycapronsäureestern und Adipinsäurediestern können noch weitere Komponenten enthalten, die einen Gewichtsanteil von bis zu 20% ausmachen können, bevorzugt aber unter 10 % Anteil liegen, besonders bevorzugt unter 5%. Diese Komponenten sind z. B. 1,5-Pentandiol, Cyclohexandiolen, ungesättigten Adipinsäurediestern, Pimelinsäurediestern, Caprolacton, 5-Hydroxycapronsäureester sowie Diestern auf Basis v. a. von 6-Hydroxycapronsäureestern.

Die Verdampfung erfolgt bei 180 bis 300 °C. Es kann vorteilhaft sein, zusätzlich ein unter den Reaktionsbedingungen inertes Lösungsmittel mitzuverdampfen. Als derartige Lösungsmittel kommen zum Beispiel Ether wie Tetrahydrofuran oder Dioxan aber auch Alkohole in Frage. Vorteilhaft verwendet man 10 bis 95 Gew.-%ige Lösungen von 6-Hydroxycapronsäureestern und Adipinsäurediestern in derartigen Lösungsmitteln als Edukt für das erfindungsgemäße Verfahren.

Inerte Trägergase sind beispielsweise Stickstoff, Kohlendioxid, Wasserstoff oder Edelgase wie zum Beispiel Argon. Vorzugsweise wird Stickstoff oder Wasserstoff als Trägergas verwendet. In der Regel verwendet man je Mol dampfförmigen 6-Hydroxycapronsäureester 5 bis 100 Mol Trägergas, bevorzugt sind 8 bis 50 Mol, besonders bevorzugt 10 bis 30 Mol. Das Trägergas wird bevorzugt mittels eines Gebläses oder eines Kompressors im Kreis geführt, wobei ein Teilstrom ausgeschleust werden und entsprechend durch Frischgas ergänzt werden kann.

Als Katalysatoren für die Herstellung von Caprolacton durch Cyclisierung von 6-Hydroxycapronsäureestern sind Aktivkohlen geeignet. Unter Aktivkohlen sind dabei Kohlenstoff enthaltende Materialien zu verstehen, die z. B. ausgehend von Holz, Sägespänen, Kokosnussschalen oder Braunkohle durch chemische oder physikalische Aktivierungsverfahren hergestellt werden können (Ullmann's Encyclopedia of Industrial Chemistry, 6. Auflage, Band 6, Seiten 326 bis 350).

Aktivkohlen sind porös, besitzen eine hohe Oberfläche und bestehen aus sehr kleinen Kristalliten mit Graphitstruktur. Die Zwischenräume zwischen den Kristalliten bestehen aus amorphem Kohlenstoff.

Für die Herstellung von Caprolacton sind Aktivkohlen in Form von Pulvern, Granulaten oder besonders bevorzugt, von Formkörpern geeignet. Ihre innere Oberfläche ist größer als 400 m²/g und beträgt im Allgemeinen 500 m²/g bis 1500 m²/g. Das verzweigte Porensystem enthält Mesoporen (d = 2 bis 50 nm), Mikroporen (d = 0,8 bis 2 nm), Submikroporen (d ≤ 0,8 nm) und Makroporen (d ≥ 50 nm).

Das Porenvolumen ist für das erfindungsgemäße Verfahren größer als 25 cm³/100 g.

An der Oberfläche der Aktivkohle können sich funktionelle Gruppen wie z. B. Carbonyl-, Carboxyl- oder Phenolgruppen befinden, die aus den Ausgangsverbindungen der Aktivkohlen stammen oder bei den Aktivierungsprozessen entstanden sind.

Die Cyclisierung der Gemische aus 6-Hydroxycapronsäureestern und 0 bis 40 % Adipinsäureester in Gegenwart von Aktivkohle als Katalysator kann in der Flüssigphase, bevorzugt aber der Gasphase erfolgen.

Die genannten Aktivkohlen können auch als Trägersubstanzen für folgende Metalloxide wie Magnesiumoxid, Zinkoxid, Bortrioxid, Titandioxid, Siliciumdioxid, Zinndioxid, Wismutoxid, Kupferoxid, Lanthanoxid, Zirkondioxid, Vanadiumoxide, Chromoxide, Wolframoxide, Eisenoxide, Ceroxid, Aluminiumoxid, Hafniumoxid, Bleioxid, Antimonoxid, Bariumoxid, Calciumoxid, Natriumhydroxid, Kaliumhydroxid und Neodymoxid dienen, die als Katalysatoren für die Cyclisierung von Gemischen aus 6-Hydroxycapronsäureestern und Adipinsäurediestern geeignet sind.

Die Aktivkohle-Katalysatoren können in der Reaktionszone fest angeordnet und das dampfförmige Gemisch aus 6-Hydroxycapronsäureestern, 0 bis 40 Gew.-% Adipinsäurediestern und Trägergasen darüber geleitet werden. Es ist jedoch auch möglich, dass sich der Katalysator in auf- und abwirbelnder Bewegung (Wirbelbett) befindet. Vorteilhaft hält man eine Katalysatorbelastung von 0,01 bis 40, bevorzugt 0,05 bis 20, insbesondere 0,07 bis 10 g Edukt (Gemisch aus 6-Hydroxycapronsäureester und ggf. Adipinsäurediester) je g Katalysator und Stunde ein.

Die Cyclisierungsreaktion kann auch in Abwesenheit eines Cyclisierungs-Katalysators gearbeitet werden. Für die Reaktion benötigt man dann jedoch höhere Temperaturen oder längere Verweilzeiten im Reaktor als bei dem erfindungsgemäßen Verfahren.

Die erfindungsgemäße Umsetzung zu Caprolacton wird bei einer Temperatur von 150 bis 450°C, vorzugsweise bei 200 bis 400 °C, insbesondere 230 bis 350 °C durchgeführt. Im Allgemeinen wird die Reaktion unter Atmosphärendruck durchgeführt. Es ist jedoch auch möglich, schwach verminderten Druck, z. B. bis zu 500 mbar oder schwach erhöhten Druck, z. B. bis zu 5 bar anzuwenden. Sofern ein fest angeordneter Katalysator verwendet wird, hat es sich als besonders günstig erwiesen, dass vor dem Katalysator ein höherer Druck eingestellt wird als nach dem Katalysator, so dass sich eventuell bildende hochsiedende Komponenten nicht oder weniger auf dem Katalysator ablagern können.

Der Reaktionsaustrag wird mit geeigneten Kühlvorrichtungen kondensiert. Wird ein fest angeordneter Katalysator verwendet, so kann der Reaktor, beispielsweise ein Schacht- oder ein Rohrbündelreaktor in auf- bzw. abwärts gerichteter Strömungsweise betrieben werden. Die Umsetzung erfolgt in mindestes einem Reaktor.

Der Reaktionsaustrag der Cyclisierung enthält als Hauptkomponente das Zielprodukt Caprolacton, weiterhin den bei der Cyclisierung freigesetzten C₁-C₆-Alkohol, ggf. Adipinsäurediester und gegebenenfalls nicht umgesetzten 6-Hydroxycapronsäureester, ggf. Oligoester und ggf. Lösungsmittel. Dieses Gemisch wird durch ein- oder mehrstufige Destillation in der Stufe 14 bei vermindertem Druck so aufgetrennt, dass Caprolacton in einer Reinheit von mindestens 99 % gewonnen wird. Bevorzugt liegt die Reinheit bei über 99,5 %, besonders bevorzugt bei über 99,8 %.

Die ein- oder mehrstufigen Destillationen zur Reinigung des Caprolactons werden bei Sumpftemperaturen von 70 bis 250 °C, bevorzugt 90 bis 230 °C, besonders bevorzugt 100 bis 210 °C und Drücken von 1 bis 500 mbar, bevorzugt 5 bis 200 mbar, besonders bevorzugt 10 bis 150 mbar durchgeführt.

Wird dafür eine Kolonne verwendet, so werden über Kopf der gegebenenfalls noch vorhandene Veresterungsalkohol, sowie andere C₁- bis C₆-Leichtsieder abgetrennt, über Seitenstrom reines Caprolacton und über den Sumpf Adipinsäurediester und gegebenenfalls noch nicht umgesetzter Hydroxycapronsäureester, der zurückgeführt wird. Der Adipinsäurediester kann, ggf. zusammen mit dimeren oder oligomeren Estern in einen Hydrierreaktor eingeschleust werden und gemäß WO 97/31883 oder DE-A-19750532 zu 1,6-Hexandiol umgesetzt werden. Wurde Methanol als Veresterungsalkohol eingesetzt, so wird über Kopf ggf. auch das Azeotrop aus Adipinsäuredimethylester und Caprolacton erhalten, das entweder in die Hydrierung zu 1,6-Hexandiol gelangen kann oder in den Prozess, beispielsweise in die Stufen nach der Veresterung zurückgeführt werden kann.

Sofern unumgesetzter 6-Hydroxycapronsäureester anfällt, wird dieser bevorzugt zur Rückgewinnung in die destillative Ester-Trennung vor der Caprolactonsynthesestufe geleitet. Es ist natürlich grundsätzlich auch möglich, ihn zusammen mit den Adipinsäurediestern in die Hydrierung zum 1,6-Hexandiol zu fahren.

Falls oligomere C₆-Ester entstehen, so können diese gemäß EP-B 1 030 827 ebenfalls in die Hydrierung zum 1,6-Hexandiol eingebracht werden.

Das erfindungsgemäße Verfahren zur Herstellung von Caprolacton in einer Reinheit über 99 % durch Cyclisierung von Gemischen aus 6-Hydroxycapronsäureestern und 0 bis 40 Gew.-% Adipinsäurediestern in Gegenwart von Aktivkohle als Katalysator oder ohne Katalysator lässt sich mit langen Katalysator-Standzeiten durchführen. Dabei werden bei hohen Ester-Umsätzen hohe Caprolacton-Selektivitäten erzielt.

Das Verfahren wird anhand der nachfolgenden Beispiele näher erläutert.

### Beispiel 1

Ein 6-Hydroxycapronsäuremethylester-Strom, der 84,0 % 6-Hydroxycapronsäuremethylester, 1,6 % 1,4-Cyclohexandiole, 1,4 % 1,5-Pentandiol, 5,0 % ungesättigte Adipinsäuredimethylester, 2,8 % Adipinsäuredimethylester, 0,2 % Pimelinsäuredimethylester, 1,6 % dimeren Ester sowie weitere Verbindungen, die mengenmäßig jeweils unter 0,1 % vorlagen, hergestellt entsprechend WO 97/31.883, enthielt, wurde bei 280 °C in einen Verdampfer gepumpt und von dort gasförmig zusammen mit ca. 800 NL Wasserstoff/h bei 280°C und Normaldruck über 3000 ml Supersorbon K (von Firma DonauCarbon, 4mm Stränge) geleitet. Der Reaktionsaustrag wurde mittels eines Wasserkühlers auskondensiert und analysiert. Die Katalysatorbelastung lag bei verschiedenen Versuchseinstellungen zwischen 0,05 und 0,09 kg Feed/Liter Katalsator *h. Insgesamt wurde über einen Zeitraum von 14 Tagen gefahren. Dabei lagen die Hydroxycapronsäureesterumsäzte bei ca. 95 bis 97 % und Caprolactonausbeuten zwischen 96 und 98 %. Als weiteres Produkt wurde dimeres Caprolacton mit 2 - 3 % Ausbeute gefunden (Prinzipiell ließe sich dieses in die Reaktion rückführen, um die Ausbeute zu erhöhen). Sowohl der gesättigte, als auch der ungesättigte Adipinsäuredimethylester passierten den Katalysator praktisch unverändert.

Die gesammelten Reaktionsausträge wurden diskontinuierlich in einer 1 m Füllkörperkolonne destilliert. Bei 10 mbar konnte Caprolacton in einer Reinheit von bis zu 99,8 % erhalten werden.

## Patentansprüche

1. Verfahren zur Herstellung von ε-Caprolacton in einer Reinheit über 99 %, **dadurch gekennzeichnet, dass** 6-Hydroxycapronsäureester-enthaltend 0 bis 40 Gew.-% Adipinsäurediester, in der Gasphase bei 150 bis 450 °C in Gegenwart von Aktivkohle als Katalysator cyclisiert und aus dem Cyclisierungsprodukt durch Destillation ε-Caprolacton gewonnen wird.

2. Verfahren nach Anspruch 1, wobei die 6-Hydroxycapronsäure 0,5 bis 40 Gew.-% Adipinsäurediester enthält.

3. Verfahren nach einem der Ansprüche 1 bis 2, wobei der 6-Hydroxycapronsäureester enthaltend 0 bis 40 Gew.-% Adipinsäurediester durch katalytische Hydrierung von Adipinsäurediestern oder Eduktströmen, die diese Ester als wesentliche Bestandteile enthalten, Destillation des Hydrieraustrags und Abtrennung des Hexandiols gewonnen wird.

4. Verfahren nach einem der Ansprüche 1 bis 3, wobei ein Adipinsäure-, 6-Hydroxycapronsäure- und bis zu 5 Gew.-% 1,4-Cyclohexandiole enthaltendes Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhältlich ist, mit einem Alkohol der 1 bis 12 C-Atome enthält zu den entsprechenden Carbonsäureestern verestert wird, das so erhaltene Veresterungsgemisch in mindestens einer Destillationsstufe so aufgetrennt wird, dass man den 0 bis 40 Gew.-% Adipinsäurediester enthaltenden 6-Hydroxycapronsäureester-Strom erhält.

5. Verfahren nach einem der Ansprüche 1 bis 4, wobei zur Herstellung des 6-Hydroxycapronsäuremethylester enthaltend 0 bis 40 Gew.-% Adipinsäuredimethylester
- das erhaltene Veresterungsgemisch in einer ersten Destillationsstufe von überschüssigem Methanol und Leichtsiedern befreit wird,
- aus dem Sumpfprodukt in einer zweiten Destillationsstufe eine Auftrennung in eine Esterfraktion mit einem Gehalt von weniger als 20 % 1,4 Cyclohexandiole bezogen auf den ursprünglichen Gehalt an 1,4-Cyclohexandiolen des Estergemischs und eine mit mehr als 80 % 1,4-Cyclohexandiole bezogen auf den ursprünglichen Gehalt an 1,4-Cyclohexandiolen des Estergemischs enthaltende Fraktion durchgeführt wird,
- der 0 bis 40 Gew.-% Adipinsäuredimethylester enthaltenden 6-Hydroxycapronsäuremethylester-Strom von der Esterfraktion in einer dritten Destillationsstufe abgetrennt wird.

6. Verfahren nach einem der Ansprüche 1 bis 5, wobei in Gegenwart eines inerten Trägergases ausgewählt aus Gruppe von Stickstoff, Kohlendioxid, Wasserstoff und Edelgasen cyclisiert wird.

7. Verfahren nach einem der Ansprüche 1 bis 6, wobei bei 150 bis 450 °C cyclisiert wird.

## Claims

1. A process for preparing ε-caprolactone in a purity of more than 99%, which comprises cyclizing 6-hydroxycaproic ester comprising from 0 to 40% by weight of adipic ester in the gas phase at from 150 to 450 °C in the presence of activated carbon as a catalyst, and obtaining ε-caprolactone from the cyclization product by distillation.

2. The process according to claim 1, wherein the 6-hydroxycaproic acid comprises from 0.5 to 40% by weight of adipic ester.

3. The process according to either of claims 1 and 2, wherein the 6-hydroxycaproic ester comprising from 0 to 40% by weight of adipic ester is obtained by catalytic hydrogenation of adipic esters or reactant streams which comprise these esters as significant constituents, distillation of the hydrogenation effluent and removal of the hexanediol.

4. The process according to any one of claims 1 to 3, wherein a carboxylic acid mixture which comprises adipic acid, 6-hydroxycaproic acid and up to 5% by weight of 1,4-cyclohexanediols and is obtainable as a by-product of the oxidation of cyclohexane to cyclohexanone/cyclohexanol with oxygen or oxygen-comprising gases by water extraction of the reaction mixture is esterified with an alcohol which comprises from 1 to 12 carbon atoms to give the corresponding carboxylic esters, and the esterification mixture thus obtained is separated in at least one distillation stage so as to obtain the 6-hydroxycaproic ester stream comprising from 0 to 40% by weight of adipic ester.

5. The process according to any one of claims 1 to 4, wherein methyl 6-hydroxycaproate comprising from 0 to 40% by weight of dimethyl adipate is prepared by
- freeing the resulting esterification mixture of excess methanol and low boilers in a first distillation stage,
- in a second distillation stage, separating the bottom product into an ester fraction having a content of less than 20% 1,4-cyclohexanediols based on the original content of 1,4-cyclohexanediols in the ester mixture and a fraction comprising more than 80% 1,4-cyclohexanediols based on the original content of 1,4-cyclohexanediols in the ester mixture,
- in a third distillation stage, removing the methyl 6-hydroxycaproate stream comprising from 0 to 40% by weight of dimethyl adipate from the ester fraction.

6. The process according to any one of claims 1 to 5, wherein cyclization is effected in the presence of an inert carrier gas selected from nitrogen, carbon dioxide, hydrogen and noble gases.

7. The process according to any one of claims 1 to 6, wherein cyclization is effected at from 150 to 450°C.

## Revendications

1. Procédé pour la production d'ε-caprolactone en une pureté de plus de 99 %, **caractérisé en ce qu'**on cyclise de l'ester d'acide 6-hydroxycaproïque contenant 0 à 40 % en poids de diester d'acide adipique, dans la phase gazeuse, à une température de 150 à 450 °C, en présence de charbon actif en tant que catalyseur, et à partir du produit de cyclisation on obtient par distillation l'ε-caprolactone.

2. Procédé selon la revendication 1, dans lequel l'acide 6-hydroxycaproïque contient 0,5 à 40 % en poids de diester d'acide adipique.

3. Procédé selon la revendication 1 ou 2, **caractérisé en ce que** l'ester d'acide 6-hydroxycaproïque contenant 0 à 40 % en poids de diester d'acide adipique est obtenu par hydrogénation catalytique de diesters d'acide adipique ou de courants de produits de départ qui contiennent ces esters en tant que composants essentiels, distillation de la décharge d'hydrogénation et séparation de l'hexanediol.

4. Procédé selon l'une quelconque des revendications 1 à 3, dans lequel on estérifie par un alcool qui contient de 1 à 12 atomes de carbone, en les esters d'acides carboxyliques correspondants, un mélange d'acides carboxyliques contenant de l'acide adipique, de l'acide hydroxycaproïque et jusqu'à 5 % en poids de 1,4-cyclohexanediols, qui peut être obtenu en tant que sous-produit de l'oxydation du cyclohexane en cyclohexanone/cyclohexanol avec de l'oxygène ou des gaz contenant de l'oxygène, par extraction à l'eau du mélange réactionnel, on fractionne dans au moins une étape de distillation le mélange d'estérification ainsi obtenu, de manière à obtenir le courant d'ester d'acide 6-hydroxycaproïque contenant 0 à 40 % en poids de diester d'acide adipique.

5. Procédé selon l'une quelconque des revendications 1 à 4, dans lequel pour la production du 6-hydroxycaproate de méthyle contenant 0 à 40 % en poids d'adipate de diméthyle
- dans une première étape de distillation on libère du méthanol en excès et de composants à bas point d'ébullition le mélange d'estérification obtenu,
- à partir du produit de pied on effectue dans une deuxième étape de distillation un fractionnement en une fraction ester ayant une teneur de moins de 20 % en 1,4-cyclohexanediols par rapport à la teneur initiale en 1,4-cyclohexanediols du mélange d'esters et une fraction contenant plus de 80 % de 1,4-cyclohexanediols par rapport à la teneur initiale en 1,4-cyclohexanediols du mélange d'esters,
- dans une troisième étape de distillation on sépare de la fraction ester le courant de 6-hydroxycaproate de méthyle contenant 0 à 40% en poids d'adipate de diméthyle.

6. Procédé selon l'une quelconque des revendications 1 à 5, dans lequel on effectue la cyclisation en présence d'un gaz vecteur inerte choisi dans le groupe constitué par l'azote, le dioxyde de carbone, l'hydrogène et les gaz rares.

7. Procédé selon l'une quelconque des revendications 1 à 6, dans lequel on effectue la cyclisation à une température de 150 à 450 °C.
